# EUROPÄISCHE PATENTANMELDUNG

(11) **EP 3 318 266 A1**
(43) Veröffentlichungstag der Anmeldung: **09.05.2018**
(21) Anmeldenummer: 16197117.1
(22) Anmeldetag: 03.11.2016
(51) Int. Cl.: A61K 38/12, A61K 45/06, A61K 31/407, A61K 31/427, A61K 31/546, A61K 31/665, A61K 31/7036, A61K 33/00, A61P 11/00, A61P 31/04

(54) **PHARMAZEUTISCHE ZUSAMMENSETZUNG ZUR ANWENDUNG BEI DER BEHANDLUNG EINER ATEMWEGSERKRANKUNG**

(71) Anmelder: Riethmüller, Joachim, 72076 Tubingen (DE)
(72) Erfinder: Riethmüller, Joachim, 72076 Tübingen (DE); Graepler-Mainka, Ute, 72070 Tübingen (DE)
(74) Vertreter: Witte, Weller & Partner Patentanwälte mbB

(57) **Zusammenfassung**

Die vorliegende Erfindung betrifft eine pharmazeutische Zusammensetzung zur Anwendung bei der Behandlung einer Atemwegserkrankung, mit mindestens zwei gegebenenfalls räumlich getrennten Wirkstoffgemischen sowie deren Verwendung. Ferner betrifft die vorliegende Erfindung ein Kit zur Anwendung bei der Behandlung einer Atemwegserkrankung.

## Beschreibung

Die vorliegende Erfindung betrifft eine pharmazeutische Zusammensetzung, die Verwendung derselben zur Behandlung einer Atemwegserkrankung sowie ein Kit zur Behandlung einer Atemwegserkrankung.

Unter dem Begriff "Atemwegserkrankungen" werden verschiedene Krankheiten zusammengefasst, die die Atemwegsorgane, beispielsweise die Lunge, betreffen. Zu den bekanntesten Atemwegserkrankungen zählen die Bronchitis, die Lungenentzündung (Pneumonie), Tuberkulose, Lungenkrebs, chronisch obstruktive Lungenerkrankung (engl. "chronic obstructive pulmonary disease", COPD), Bronchiektasien, Asthma, Schlafapnoe, Lungenemphysem, Primäre ciliäre Dyskinesie (PCD) und cystische Fibrose (engl. "cystic fibrosis", CF).

Da Atemwegserkrankungen nach wie vor zu den häufigsten Todesursachen zählen, ist die Entwicklung von neuartigen Behandlungskonzepten ein dringendes Bedürfnis und stellt eine Herausforderung an die moderne Medizin dar. Insbesondere chronisch bakterielle Lungenerkrankungen, wie sie bei Patienten mit COPD oder CF auftreten, stehen im Fokus der derzeitigen Forschung.

COPD bezeichnet nicht eine einzelne Krankheit, sondern steht als Sammelbegriff für eine chronische progressive Störung des Lungenparenchyms. Gemäß der WHO-Definition liegt eine COPD vor, wenn Husten und Auswurf über wenigstens drei Monate in mindestens zwei aufeinanderfolgenden Jahren bestehen.

Gemäß den jüngsten WHO-Schätzungen leiden derzeit ca. 64 Mio. Menschen weltweit an COPD. Ferner ist COPD gegenwärtig die vierthäufigste Todesursache. Für die nächsten Jahrzehnte ist ein weiterer Anstieg von Prävalenz, Morbidität und Mortalität zu erwarten.

Der Krankheitsverlauf der COPD ist durch eine progrediente Verschlechterung der Lungenfunktion und eine zunehmende Beeinträchtigung des Befindens, der Leistungsfähigkeit und der Lebensqualität gekennzeichnet, insbesondere hervorgerufen durch rezidivierende Exazerbationen und zunehmende Auswirkungen auf andere Organe.

Besonders die progrediente Lungenschädigung begünstigt eine bakterielle Lungeninfektion. Bei annähernd der Hälfte der Patienten mit einer akuten Exazerbation der COPD (AECOPD) besteht die Ursache in einer bakteriellen Infektion. Die häufigsten Erreger sind: nicht typisierbare *H. influenzae* (NTHI), *S. pneumoniae, M. catarrhalis, Enterobacteriaceae, S. pneumoniae* und *P. aeruginosa*.

Auch bei der Erbkrankheit CF führen sekundär erworbene chronisch bakterielle Lungeninfektionen zu einer verminderten Lebenserwartung. CF ist die häufigste angeborene Stoffwechselkrankheit der kaukasischen Bevölkerung. Sie geht mit einer Störung des Chloridionenkanals (CPTR) einher und verursacht ein extrem zähes Sekret aller exokrinen Drüsen. In Deutschland leben rund 8.000 Patienten mit dieser bis dato unheilbaren Krankheit und in Europa sind es rund 50.000 Patienten.

Die häufigste Ursache für die Morbidität und Mortalität bei Patienten mit CF ist die akute und chronische bakterielle Besiedlung des Bronchialsystems mit rezidivierenden bronchopulmonalen Infektionen, den sog. Exazerbationen. Diese treiben den zystischen Umbau der Lunge voran und führen letztendlich zum terminalen Lungenversagen und zum Tode. Aus diesem Grund spielen die Infektionen und die daraus resultierende Entzündung eine sehr wichtige Rolle bei der Behandlung der CF.

Sowohl bei COPD- als auch bei CF-Patienten führen die Besiedlung und die Infektionen der Lunge mit Problemkeimen, wie beispielsweise multiresistenten Keimen, sofern diese unbehandelt bleiben, zu einem frühzeitigen Tod. Derzeitige Behandlungsstrategien für diese Patienten sollen durch konsequente antibiotische Therapien, sowohl systemischer als auch inhalativer Art, die Lebensqualität verbessern. Durch den regelmäßigen Einsatz von Standardantibiotika kommt es häufig zu einer Resistenzentwicklung der Bakterien gegenüber den Antibiotika. Die Entstehung von multiresistenten Erregern, wie beispielsweise *P. aeroginosa* oder *MRSA,* sind die Folge. Sie stellen zunehmend ein Behandlungsproblem dar.

Eine alternative Behandlungsmethode betrifft die Verabreichung von gasförmigem Stickstoffmonoxid (NO). NO wird bis dato in der Therapie der pulmonalen Hypertonie und des ARDS (Acute Respiratory Distress Syndrome) eingesetzt. Dabei soll NO, in hohen Konzentrationen die Erreger unabhängig von deren Empfindlichkeit/ Resistenz abzutöten. Diese therapeutische Behandlung wurde bereits in einer Phase-I-Studie bei Patienten, die unter einer Atemwegserkrankung leiden, eingesetzt (Deppisch, C., Herrmann, G., Graepler-Mainka, U. et al. Infection (2016) 44: 513). In dieser Studie zeigte sich, dass sowohl die Keimzahlreduzierung, als auch die Lungenfunktionsverbesserung und auch die rheologische Verbesserung des Sputums erreicht werden konnte.

Problematisch jedoch ist, dass sowohl die antibiotische als auch die NO-Therapie die Lungenfunktion nur kurzfristig verbessern, beziehungsweise nur kurzfristig eine weitere Verschlechterung der Lungenfunktion vermeiden können. Zudem lieferten die beiden Therapieformen gerade bei Patienten mit einem zunehmenden Schweregrad der Krankheit (gemessen am FEV₁-Wert) keine zufriedenstellenden Resultate.

Vor diesem Hintergrund ist es die der Erfindung zugrundeliegende Aufgabe, eine pharmazeutische Zusammensetzung bereitzustellen, mit der die vorstehend genannten Nachteile aus dem Stand der Technik vermindert, vorzugsweise vermieden werden.

Diese Aufgabe wird durch eine pharmazeutische Zusammensetzung zur Anwendung bei der Behandlung einer Atemwegserkrankung gelöst, mit mindestens zwei gegebenenfalls räumlich getrennten Wirkstoffgemischen, wobei das erste Wirkstoffgemisch zumindest drei Antibiotika aufweist und das zweite Wirkstoffgemisch zumindest gasförmiges NO aufweist.

Die Aufgabe wird ferner auch durch die Verwendung einer pharmazeutischen Zusammensetzung mit mindestens zwei gegebenenfalls räumlich getrennten Wirkstoffgemischen zur Behandlung einer Atemwegserkrankung gelöst, wobei das erste Wirkstoffgemisch zumindest drei Antibiotika aufweist und das zweite Wirkstoffgemisch zumindest gasförmiges NO aufweist.

Ferner wird die Aufgabe erfindungsgemäß durch ein Kit zur Behandlung einer Atemwegserkrankung gelöst, welches ein erstes Wirkstoffgemisch von zumindest drei Antibiotika und ein zweites Wirkstoffgemisch von zumindest gasförmigen NO aufweist.

Die vorliegende Erfindung betrifft eine neuartige Kombination von verschiedenen Wirkstoffgemischen, die zusammen erstmalig durch die Erfinder in der Behandlung von Atemwegserkrankungen eingesetzt werden. Von besonderer Bedeutung ist die Anwendung bei solchen Atemwegserkrankungen, welche mit einer progredienten Lungenschädigung einhergehen.

In klinischen Studien konnten die Erfinder zeigen, dass bei der Behandlung von Atemwegserkrankungen unter Zuhilfenahme der Kombination des ersten und zweiten Wirkstoffgemisches nicht nur ein additiver sondern vielmehr ein synergistischer Effekt erzielt werden konnte. Dieser Synergismus war vor allem deshalb überraschend, da die isolierte Behandlung mit den einzelnen Wirkstoffgemischen nicht die gewünschten Erfolge zeigte.

Ein Fachmann hätte allenfalls einen additiven Effekt durch die Kombination erwartet, nicht aber einen derartigen Synergismus. Ferner hätte der Fachmann eine massive Zunahme von Nebenwirkungen vermutet und daher keine Anstrengung unternommen, die beiden Therapieformen zu kombinieren.

Überraschenderweise zeigte sich, dass die erfindungsgemäße pharmazeutische Zusammensetzung von Patienten sehr gut toleriert wurde. Ferner konnte auch ein langanhaltender positiver Effekt nachgewiesen werden. So zeigten die Patienten nicht nur einen verbesserten Lungenfunktionswert, auch die typischen Symptome der chronischen Entzündung im Sinne einer neuerlichen Lungenfunktionsverschlechterung und/oder pulmonalen Exazerbation traten erst deutlich verzögert auf. Die neue Kombination der verschiedenen Wirkstoffgemische bietet eine geeignete Therapie, die für eine klinische Implementierung geeignet ist.

Ferner haben die Erfinder herausgefunden, dass die pharmazeutische Zusammensetzung auch wirkungsvoll bei Patienten ist, die mittels den herkömmlichen Therapien keine Lungenfunktionsverbesserung mehr zu erwarten haben. Es ist den Erfindern damit gelungen auch für diese Patientengruppen mit einem FEV₁ Wert von <50% erstmalig eine geeignete und zudem besonders wirkungsvolle Therapiebehandlung bereitzustellen. Bei repetitiver Anwendung kann die Lungenfunktion nachhaltig verbessert werden, was somit zu einer verbesserten Lebenserwartung dieser Patientengruppe führt.

Erfindungsgemäß wird unter "Wirkstoffgemisch" ein therapeutisch wirksames Gemenge aus arzneimittelwirksamen Bestandteilen verstanden. Mindestens zwei gegebenenfalls räumlich getrennte Wirkstoffgemische bilden zusammen die erfindungsgemäße pharmazeutische Zusammensetzung. Das erste der beiden Wirkstoffgemische enthält als arzneimittelwirksame Bestandteile zumindest drei Antibiotika. Das zweite Wirkstoffgemisch enthält als arzneimittelwirksamen Bestandteil zumindest gasförmiges NO. Es versteht sich jedoch, dass weitere arzneimittelwirksame Bestandteile in den Wirkstoffgemischen enthalten sein können, beispielsweise solche, die die erfindungsgemäße Wirksamkeit verstärken; Beispiele hierfür sind: Antimykotika, Chloridsalzlösungen etc.

Im Folgenden werden die Begriffe "arzneimittelwirksamer Bestandteil" und "Wirkstoff" synonym verwendet.

Die Wahl der jeweiligen Konzentrationen der Wirkstoffe in den Wirkstoffgemischen wird von dem behandelnden Fachmann vorgenommen. Sie richtet sich unter anderem nach der Indikation, dem Zustand des zu behandelnden Patienten, dem Verabreichungsweg und den toxikologischen Parametern.

Erfindungsgemäß wird unter "gegebenenfalls räumlich getrennten Wirkstoffgemischen" verstanden, dass die beiden Wirkstoffgemische in getrennten Arzneimittelformulierungen vorliegen können. Diese können gegebenenfalls getrennt voneinander verabreicht werden. Dies hat zum einen den Vorteil, dass die beiden Wirkstoffgemische zeitlich unabhängig voneinander verabreicht werden können. Zum anderen hat es den Vorteil, dass die Applikation des zweiten Wirkstoffgemisches bevorzugt inhalativ durchgeführt wird, während die Verabreichung des ersten Wirkstoffgemisches bevorzugt oral oder invasiv erfolgen kann.

Erfindungsgemäß können auch in dem ersten Wirkstoffgemisch die zumindest drei Antibiotika in mehreren bzw. unterschiedlichen Formulierungen vorliegen. Dies hat den Vorteil, dass die Wirkstoffe je nach Beschaffenheit auf unterschiedliche Art und Weise einzeln oder zusammen verabreicht werden können. So können beispielsweise die zumindest drei Antibiotika des ersten Wirkstoffgemisches dem Patienten oral, intravenös und/oder inhalativ verabreicht werden.

Erfindungsgemäß können die drei Antibiotika des ersten Wirkstoffgemisches jegliche auf dem Markt verfügbare Antibiotika betreffen. Ein Fachmann ist in der Lage, eine geeignete Kombination zu wählen, die in einem erweiterten Wirkungsspektrum, einer synergistischen Wirkung, einer Verzögerung von Resistenzentwicklungen, einer Inaktivierung von β-Laktamasen und/oder einer Synthesehemmung bakterieller Toxine resultiert.

In dem ersten Wirkstoffgemisch können bevorzugte Kombinationen von Antibiotika erfindungsgemäß folgende sein: Kombination eines Cephalosporin (bspw. Cefazidim, Cefepim, Cefesulodin) und einem Aminogycosid (bspw. Tobramycin, Amikacin, Gentamicin, Netilmicin) mit einem beliebigen dritten Antibiotikum, gegebenenfalls einem Polymyxin (bspw. Colistin). Eine weitere bevorzugte Kombination könnte aus einem Carbapenem (bspw. Meropenem, Imipenem), einem Aminoglycosid und einem beliebigen weiteren Antibiotikum, gegebenenfalls eines Polymyxin- Antibiotikum, bestehen. Eine weitere bevorzugte Kombination könnte folgende sein: Penicillin (bspw. Azlocillin, Piperacillin in Verbindung mit Tazobactam, Ticarcillin) oder ein Epoxid-Antibiotika (bspw. Fosfomycin) mit einem Aminoglycosid und einem beliebigen weiteren Antibiotikum. Alternativ können Floxacine (bspw. Ciprofloxacin und Levofloxacin) kombiniert werden mit einem Aminoglycosid und mit einem beliebigen weiteren Antibiotikum.

Eine alternative bevorzugte Kombination kann aus einem Aminoglycosid (bspw. Tobramycin) und einem Polymyxin (bspw. Colistin) mit einem beliebigen dritten Antibiotikum bestehen. Eine weitere bevorzugte Kombination kann aus einem Carbapenem (bspw. Meropenem) einem Aminoglycosid (bspw. Tobramycin) und einem Polymyxin (bspw. Colistin) bestehen. Weitere bevorzugte Kombinationen sind in der Patentanmeldung DE 10 2011 108 227 A1 offenbart und können erfindungsgemäß in dem ersten Wirkstoffgemisch verwendet werden. Der Inhalt der genannten Anmeldung ist durch Inbezugnahme Bestandteil der vorliegenden Anmeldung.

Bei der antibiotischen Behandlung von Atemwegserkrankungen wird im Stand der Technik zwischen der Exazerbationstherapie und der prophylaktischen Dauertherapie unterschieden. Die prophylaktische Dauertherapie zielt meist auf den Erreger *P. aeruginosa* ab. Hierbei werden die Antibiotika unabhängig von Symptomen und Erregernachweis verabreicht. Die Anwendung der Exazerbationstherapie ist an Symptome gekoppelt und wird daher an das Antibiogramm und den spezifischen Erregernachweis angeglichen. Typische Erreger hierbei sind beispielsweise: S. *aureus, H. influenza, P. aeruginosa*, *B. cepacia, S. maltophilia.* Die beiden Therapien werden im Stand der Technik nicht gleichzeitig angewendet. Sofern eine Exazerbationstherapie durchgeführt wird, wird die Dauertherapie klassischerweise für diesen Zeitraum unterbrochen und im Anschluss wieder aufgenommen (ON/OFF Therapie).

Dagegen kann das erste Wirkstoffgemisch der pharmazeutischen Zusammensetzung erfindungsgemäß im Rahmen einer Exazerbationstherapie und/oder einer prophylaktischen Dauertherapie verwendet werden. Dies hat den Vorteil, dass erstmalig beide Therapien kombiniert und zusammen mit dem zweiten Wirkstoffgemisch bei der Behandlung von Atemwegserkrankungen eingesetzt werden können.

Die Verwendung der pharmazeutischen Zusammensetzung zur Behandlung von Atemwegserkrankungen hat ferner den Vorteil, dass zumindest drei bislang nur als Monopräparate oder als Kombinationspräparate bestehend aus zwei Antibiotika eingesetzte und sich bewehrte Antibiotika nunmehr erstmalig in Kombination mit gasförmigem NO Verwendung finden, und aufgrund einer von den Erfindern erkannten synergistischen Wirkung besonders zur Anwendung bei der Behandlung einer Atemwegserkrankung geeignet sind.

Nach einer bevorzugten Weiterbildung der pharmazeutischen Zusammensetzung sind die Antibiotika des ersten Wirkstoffgemisches ausgewählt aus der Gruppe bestehend aus: Cephalosporine, Aminoglycoside, Makrolide, Polymyxine, Peneme, Carbapenem, Penicilline, Epoxyd-Antibiotika, einschließlich Chinolone.

Cephalosporine bilden eine Gruppe von Breitband-Antibiotika und gehören zu den β-Lactamen. Die Wirkungsweise dieser Antibiotika beruht auf der Hemmung des Neuaufbaus der Zellwände von sich teilenden Bakterien, insbesondere der inneren Quervernetzung der Murinschicht. Cephalosporine sind besonders wirksam gegenüber *Staphylokokken.* Zudem weisen einige ihrer Vertreter eine erhöhte Resistenz gegen β-Lactamasen auf. Erfindungsgemäß können beispielsweise Ceftazidim, Cefepim und Cefsulodin als Vertreter dieser Gruppe zum Einsatz kommen.

Aminoglycoside beziehungsweise Aminoglycosid-Antibiotika zählen zur Gruppe der Oligosaccharid-Antibiotika. Aufgebaut sind diese aus mehreren Zucker- oder Aminozucker-Molekülen, die untereinander glykosidisch und meist zudem mit einem Aglycon verknüpft sind. Als Aglycon findet man beispielsweise ein mit Amino-Gruppen substituiertes Cyclitol.

Aminoglycoside bilden eine große Gruppe von ca. 200 wasserlöslichen Antibiotika. Sie wirken stark bakterizid durch die Hemmung der Proteinbiosynthese auf proliferierende und ruhende Erreger, indem sie an die 30-S-Untereinheit der Ribosom ankoppeln und Ablesefehler der mRNA verursachen. Dadurch werden fehlerhafte Proteine gebildet, die vielfach nicht mehr ihrer Funktion nachgehen können. In der Konsequenz werden beispielsweise defekte Proteine in die Zellmembran des Bakteriums eingebaut, was zur Lyse des Erregers führt. Erfindungsgemäß kann das erste Wirkstoffgemisch beispielsweise Amikacin, Apramycin, Geneticin (G418), Gentamicin, Kanamycin, Netilmycin, Neomycin, Paromomycin, Spectinomycin, Streptomycin und/oder Tobramycin als Vertreter dieser Gruppe aufweisen.

Makrolide beziehungsweise Makrolid-Antibiotika gehören zu der Gruppe der organisch chemisch ringförmigen Moleküle. Makrolide können eine Ringgröße von 8 bis 62 Ringgliedern aufweisen. Der Wirkungsort der Makrolid-Antibiotika ist die 50-S-Untereinheit des bakteriellen 70-S-Ribosoms. Dabei behindern sie den Syntheseprozess von Proteinen während der Elongationsphase. Die Translokation der Peptidyl-tRNA von der Akzeptorstelle zur Donorstelle wird blockiert. Dies führt zum Abbruch der Peptidsynthese und zur Freisetzung eines unfertigen Polypeptids. Hieraus resultiert eine bakteriostatische Wirkung. Bevorzugt können beispielsweise Erythromycin, Clarithromycin, Azithromycin, Roxithromycin, Josamycin, Steprimamycin und/oder Ontylosin als Vertreter dieser Gruppe im ersten Wirkstoffgemisch enthalten sein.

Polymyxine beziehungsweise Polypeptid-Antibiotika sind cyclische Heptapeptide mit einem Tripeptid als Seitenkette, an deren Ende sich eine verzweigte Fettsäure (6-Methylheptansäure) befindet. Ähnlich wie die Zellmembran besitzen sie eine Polarität, die durch die in ihnen enthaltenen Aminosäuren und endständigen hydrophoben Fettsäuren entsteht. Dadurch werden diese in die Zellmembran gramnegativer Bakterien eingelagert und stören deren Permeabilität, indem sie die osmotische Barriere aufheben. Aufgrund dessen besitzen diese Antibiotika eine bakterizide Wirkung vorwiegend gegenüber gramnegativen Bakterien, wie *Pseudomonas, E. coli, Salmonella, Shigella* etc. Erfindungsgemäß können beispielsweise Polymyxin B und/oder Colistin (Polymyxin E) verwendet werden.

Peneme bzw. Penem-Antibiotika zählen zu der Gruppe der β-Lactam-Antibiotika, deren Penem-Grundgerüst in 2,3-Position ungesättigt ist. Die Peneme sind synthetisch hergestellte Produkte und besitzen im Gegensatz zu Carbapenemen im Fünfring ein Schwefelatom. Aus dieser Gruppe kann erfindungsgemäß beispielsweise Faropenem zum Einsatz kommen.

Carbapeneme, ursprünglich als Thienamycine bezeichnet, sind sog. β-Lactam-Antibiotika mit breitem Wirkungsspektrum gegenüber fast allen pathogenen grampositiven und gramnegativen Bakterien und vielen an Mischinfektionen beteiligten Anaerobiern. Im Gegensatz zu anderen β-Lactam-Antibiotika weisen sie im Fünfring des β-Lactams ein Kohlenstoff statt eines Schwefelatoms auf. Carbapeneme hemmen die Zellwandsynthese der Bakterien durch Bindung an Penicillinbindeproteine. Erfindungsgemäß können beispielsweise Imipenem, Ertapenem, Meropenem und/oder Doripenem eingesetzt werden.

Auch Penicilline gehören zur Gruppe der β-Lactam-Antibiotika. Sie leiten sich strukturell von der 6-Aminopenicillinsäure ab, einem bizyklischen Dipeptid aus L-Cystein und L-Valin. Penicilline wirken bei der Zellteilung der Bakterien bakterizid, und zwar beim Neuaufbau der Zellwand, indem sie in die Synthese der Zellwand eingreifen, dort die innere Vernetzung verhindern und damit die Zellwand so schwächen, dass diese bei Belastung aufbricht. Insbesondere grampositive Bakterien, die sich teilen, sterben unter Penicillin-Einfluss. Erfindungsgemäß können beispielsweise Apalcillin, Azlocillin, Carbenicillin, Carnindacillin, Mezlocillin, Piperacillin in Verbindung mit Tazobactam und/oder Ticarcillin eingesetzt werden. Diese Antibiotika sind insbesondere gegenüber *P. aerugino*sa wirksam.

Ein wichtiger Vertreter der Epoxyd-Antibiotika ist das Fosfomycin, welches erfindungsgemäß eingesetzt werden kann. Dieses weist eine Phosphorsäure sowie ein Epoxid auf. Fosfomycin wirkt, indem es die Mureinbiosynthese bei Bakterien durch irreversible Hemmung der UDP-Acetylglucosamin-enolpyruvyl-transferase verhindert. Fosfomycin hat ein breites Wirkspektrum gegenüber *Staphylokokken, Streptokokken, E. coli, Enterobacter, Proteus, P. aeruginosa*, *Neisseria, H. influenzae, Citrobacter* und *Serratia.*

Chinolone bzw. Chinolon-Antibiotika leiten sich strukturell von Chinolin ab, welches am stickstoffhaltigen Ringsystem eine Carbonylgruppe sowie eine Carbonsäuregruppe trägt. Die einzelnen Chinolone unterscheiden sich bezüglich ihres Grundgerüsts und besonders in den Substituenten. Chinolone wirken an der DNA-Topoisomerase Typ II (Gyrase) und der DNA-Topoisomerase Typ IV. Dadurch wird die Spiralisierung der DNA verhindert, was sukzessiv die Chromosomlänge der Bakterien-DNA steigert. Die DNA kann schließlich nicht mehr korrekt abgelesen und repliziert werden, was zu einer verminderten Syntheseleistung der Bakterien führt. Erfindungsgemäß können beispielsweise Fluorchinolon wie Ciprofloxacin, Floxacine und/oder Levofloxacin eingesetzt werden.

Die Kombination der drei Antibiotika ausgewählt aus der oben genannten Gruppe bietet den Vorteil, dass diese Antibiotika im Markt verfügbar sind, pharmakologisch und toxikologisch umfassend getestet sind, so dass von einem beschleunigten Zulassungsverfahren ausgegangen werden kann. Viele dieser Antibiotika werden ferner bereits bei der Behandlung von Atemwegserkrankungen eingesetzt, so dass hier auf einen breiten Erfahrungsschatz bezüglich der Wirksamkeit, möglicher Nebenwirkungen etc. zurückgegriffen werden kann.

Diese Ausführungsform bietet mit der großen Auswahl an möglichen Antibiotika außerdem den Vorteil, dass eine geeignete Kombination von zumindest drei Antibiotika für jeden Patienten individuell angepasst werden kann. Ferner kann hierdurch einer möglichen Resistenzbildung besser entgegengewirkt werden.

Nach einer weiteren bevorzugten Ausgestaltung der erfindungsgemäßen pharmazeutischen Zusammensetzung ist zumindest ein Antibiotikum des ersten Wirkstoffgemisches ein Aminoglycosid, wobei dieses bevorzugt Tobramycin ist.

Aminoglycoside bzw. Aminoglycosid-Antibiotika besitzen den Vorteil, dass sie ein breites Wirkungsspektrum aufweisen und gegen viele Infektionen verwendet werden können. Das Wirkungsspektrum umfasst in erster Linie die grammnegativen *Enterobakterien* und *P. aeruginosa* sowie die grammpositiven *Staphylokokken.* Aminoglycoside können kombiniert werden mit β-Lactam-Antibiotika, mit dem Resultat eines Synergismus der Wirkung, beispielsweise bei Infektionen durch *P. aeruginosa*.

Gentamicin ist ein Antibiotikum der Aminoglycoside, welches erfindungsgemäß im ersten Wirkstoffgemisch bevorzugt enthalten sein kann. Die Behandlung von Atemwegserkrankungen mit Gentamicin zeigt gute Wirksamkeiten, vor allem gegenüber gramnegativen Erregern wie beispielsweise *P. aeruginosa*. Durch die Verwendung dieses Antibiotikums kann somit die Exazerbationsrate gesenkt und damit die Lebensqualität der behandelnden Patienten verbessert werden.

Tobramycin als Vertreter der Aminoglycoside kann erfindungsgemäß bei der Behandlung von Patienten mit Atemwegserkrankungen eingesetzt werden. Derzeit wird es in so genannten 4-wöchigen ON/OFF-Zyklen in Form einer vorbeugenden Dauertherapie verordnet. Dabei inhalieren die Patienten vier Wochen das Medikament und dann folgt eine 4-wöchige Pause. Alternativ kann Tobramycin auch invasiv verabreicht werden.

Eine ON/OFF Therapie mit Tobramycin kann die FEV₁ (Einsekundenkapazität; forciertes exspiratorisches Volumen in der ersten Sekunde) verbessern. Wie die Erfinder feststellen konnten, zeigt sich eine signifikante Verbesserung der FEV₁ Werte erst bei der Gabe von Tobramycin in Verbindung mit zwei weiteren Antibiotika sowie gasförmigem NO. Diese Verbesserung des FEV₁ geht weit über die Verbesserung des FEV₁ bei einer Monotherapie hinaus. Es zeigt sich ferner eine nachhaltige Verbesserung. Diese Maßnahme hat daher den Vorteil, dass zumindest drei Antibiotika zum Einsatz kommen, welche nach Erkenntnissen der Erfinder zu einer Verbesserung der Lungenfunktion führen.

Auch Amikacin als Vertreter der Aminoglycosid-Antibiotika kann eine Verbesserung des FEV₁ und eine Abnahme der Anzahl von *P. aeruginosa* im Sputum aufzeigen.

Die Verwendung dieser Antibiotika hat daher den Vorteil, dass die erfindungsgemäße pharmazeutische Zusammensetzung und deren erstes Wirkstoffgemisch Antibiotika aufweist, die in einer Monotherapie oder in Kombinationstherapie bestehend aus zwei Antibiotika keine ausreichende Verbesserung der FEV₁ Werte zeigten und nun aber durch geeignete Kombination mit einem bzw. zwei weiteren Antibiotika und gasförmigem NO eine synergistische Wirkung haben.

Nach einer bevorzugten Ausgestaltung ist das erste Wirkstoffgemisch der pharmazeutischen Zusammensetzung für eine Applikation im Wege einer intravenösen und/oder inhalativen und/oder oralen Verabreichung ausgestaltet. Das erste Wirkstoffgemisch wird nach dieser bevorzugten Ausgestaltung intravenös und/oder inhalativ und/oder oral verabreicht.

Diese Maßnahme hat den Vorteil, dass die Antibiotika je nach Beschaffenheit und optimaler Wirkungsweise verabreicht werden.

Der Vorteil einer intravenösen Verabreichung führt dazu, dass sich das erste Wirkstoffgemisch sofort im Blutkreislauf befindet. Intravenös dargebotene Wirkstoffgemische können zudem niedriger dosiert werden. Außerdem können intravenös verabreichte Wirkstoffgemische besser an die Bedürfnisse jedes einzelnen Patienten angepasst werden. Gerade bei Patienten mit chronischen oder langwierigen Erkrankungen bietet eine Infusion eine geeignete Darreichungsform.

Eine inhalative Verabreichung hat den Vorteil, dass die Antibiotika direkt an ihren Wirkort, nämlich in die Atemwege gelangen und Nebenwirkungen sowie geringere Antibiotikakonzentrationen in den Atemwegen, die bei systemischer Verabreichung auftreten würden, vermieden werden. Die Wirkung der erfindungsgemäßen Zusammensetzung kann dadurch optimiert werden. Eine verlängerte oder reguläre invasive Therapie kann durch einen peripher eingeführten zentralen Katheter (PICC-Leitung) oder einen sog. "Porth-a-Cath" erfolgen, um eine häufige intravenöse Behandlung zu vereinfachen.

Eine orale Verabreichung des ersten Wirkstoffgemisches hat den Vorteil, dass diese ohne Zuhilfenahme eines medizinisch ausgebildeten Personals durchgeführt werden kann. So kann der Patient gemäß einem Verabreichungsplan das erste Wirkstoffgemisch selbstständig einnehmen.

Erfindungsgemäß können die drei Antibiotika des ersten Wirkstoffgemisches zusammen verabreicht werden oder jeweils einzeln intravenös und/oder inhalativ und/oder oral. Je nach eingesetztem Antibiotikum kann so der Wirkungseintritt optimiert werden.

Nach einer weiteren bevorzugten Ausgestaltung der erfindungsgemäßen Zusammensetzung ist das zweite Wirkstoffgemisch für eine Applikation im Wege einer Inhalation ausgestaltet. Das zweite Wirkstoffgemisch wird nach dieser bevorzugten Ausgestaltung inhalativ verabreicht.

Diese Maßnahme hat den Vorteil, ähnlich wie die inhalative Verabreichung des ersten Wirkstoffgemisches, dass die Wirkstoffe direkt an ihren Wirkort, nämlich in die Atemwege gelangen. Stickstoffmonoxid wirkt gegen eine große Zahl von Bakterien und Pilzen. Die zu erwartenden Nebenwirkungen bei der Inhalation von NO-Konzentration sind dabei minimal.

Nach einer bevorzugten Weiterentwicklung der erfindungsgemäßen pharmazeutischen Zusammensetzung wird das gasförmige NO des zweiten Wirkstoffgemisches inhalativ in einer Konzentration von ca. 120 bis ca. 180 ppm, vorzugsweise von ca. 140 bis ca. 170 ppm, insbesondere ca. 160 ppm verabreicht.

Diese Maßnahme hat den Vorteil, dass eine NO-Konzentration zum Einsatz kommt, welche für den Menschen nicht toxisch ist und kaum Nebenwirkungen bei der Behandlung zu erwarten lässt.

Das zweite Wirkstoffgemisch kann erfindungsgemäß neben dem gasförmigen NO auch Sauerstoff aufweisen, so dass einem Patienten gasförmiges NO in Kombination mit Sauerstoff verabreicht werden kann. Diese Maßnahme hat den Vorteil, dass die Bindung von NO an Hämoglobin, so dass MetHb (Methämoglobin) gebildet wird, welches zu einer Abnahme des Sauerstofftransportes innerhalb der Blutgefäße führen kann, vermindert wird.

Nach einer weiteren bevorzugten Weiterentwicklung der erfindungsgemäßen pharmazeutischen Zusammensetzung ist die Atemwegserkrankung ausgewählt aus der Gruppe bestehend aus: Cystischer Fibrose (CF), chronisch obstruktiver Bronchitis (COPD), non-CF Bronchiektasien, Ventilator-assoziierte Pneumonie (VAP), Bronchiolitis, Pharyngitis, Laryngitis, Tracheitis, Pneumonie, Sinusitis, Asthma, und primäre ciliäre Dyskinesie (PCD).

Diese Maßnahme hat den Vorteil, dass eine pharmazeutische Zusammensetzung bereitgestellt wird, welche bei einigen der relevantesten Atemwegserkrankungen nutzbringend eingesetzt werden kann. So konnten die Erfinder insbesondere bei CF-Patienten, die erfindungsgemäß behandelt wurden, eine signifikante Reduzierung der Anzahl verschiedenster pathogener Keime feststellen.

Diese Maßnahme hat ferner den Vorteil, dass eine neue Therapieform für die oben genannten Atemwegserkrankungen bereitgestellt werden kann, die zu einer deutlichen Verbesserung der Lungenfunktion führt und weniger Nebenwirkungen aufweist, als die derzeitig im Stand der Technik bekannten Therapiemaßnahmen.

Nach einer weiteren bevorzugten Ausgestaltung der erfindungsgemäßen pharmazeutischen Zusammensetzung ist die Atemwegserkrankung eine bakterielle, eine mykobakterielle oder fungizide Atemwegserkrankung.

Gerade bei Patienten mit Atemwegserkrankungen kommt es vermehrt zu einer Infektion mit pathogenen Keimen wie beispielsweise Bakterien, Viren oder Pilze. Diese Maßnahme ist daher besonders vorteilhaft.

Pilzinfektionen der Atemwegsorgane führen häufig zu einer allergischen bronchopulmonalen Aspergillose (ABPA), einer Mykotoxikose oder zu einer invasiven Aspergillose. Auch diese Erkrankungen können durch die erfindungsgemäße pharmazeutische Zusammensetzung vermieden beziehungsweise behandelt werden.

Nach einer weiteren Ausgestaltung ist es bevorzugt, wenn die bakterielle Atemwegserkrankung Bakterien involviert, die ausgewählt sind aus der Gruppe bestehend aus: *Pseudomonas aeruginosa*, multiresistenter *Staphylococcus aureus, Staphylococcus aureus, Haemophilus influenzae* (HI) b, nicht-typisierbarer HI (NTHI), *Moraxella catarrhalis, Streptococcus pneumonia, Burkholderia cepacia, Acinetobacter baumanii,* strikt-anaerobe Bakterien, einschließlich *Prevotella spp.*

Die Erfinder konnten feststellen, dass die erfindungsgemäße pharmazeutische Zusammensetzung bei den vorstehend genannten Bakterien, die sehr häufig im Zusammenhang mit Atemwegserkrankungen auftreten, eine positive Wirkung zeigt. Im Gegensatz zu den derzeit durchgeführten Therapien, die im Wesentlichen auf die Behandlung von *P. aeruginosa* ausgerichtet sind, ist die erfindungsgemäße pharmazeutische Zusammensetzung durch ein deutlich breiteres Wirkspektrum gekennzeichnet und ermöglicht folglich die Behandlung auch solcher Atemwegserkrankungen, die mit herkömmlichen Therapien nicht behandelbar sind. Ferner lassen sich durch die erfindungsgemäße pharmazeutische Zusammensetzung unterschiedliche Bakterienstämme in einer Behandlung eliminieren.

Nach einer weiteren bevorzugten Ausgestaltung der erfindungsgemäßen pharmazeutischen Zusammensetzung erfolgt die Behandlung einer Atemwegserkrankung an Patienten mit eingeschränkter Lungenfunktion mit einem FEV₁ Wert < 80%, vorzugsweise < 50%, insbesondere < 40%.

Die Maßnahme hat den Vorteil, dass Patienten behandelt werden können, denen derzeit aufgrund ihrer eingeschränkten Lungenfunktion keine erfolgsversprechende Therapie zur Verfügung steht. Bei Patienten mit eingeschränkter Lungenfunktion konnten die Erfinder zeigen, dass eine isolierte Antibiotikabehandlung bzw. eine isolierte NO-Behandlung zu keiner dauerhaft signifikanten Lungenfunktionsverbesserung führt. Durch die erfindungsgemäße Therapie konnte die Lungenfunktion hingegen dauerhaft signifikant verbessert werden, so dass diese Maßnahme eine Verbesserung der Lebensqualität dieser Patientengruppen darstellt.

Nach einer bevorzugten Weiterbildung der Erfindung wird die pharmazeutische Zusammensetzung nach einem Behandlungsplan verabreicht, der einen oder mehrere Zyklen umfasst, wobei jeder Zyklus aus den folgenden Schritten besteht:
(a) mindestens einmal tägliche Verabreichung des ersten Wirkstoffgemisches in einer therapeutisch wirksamen Dosierung, wobei die Verabreichung inhalativ, oral und/oder invasiv durchgeführt wird, und
(b) zwei- bis dreimal tägliche inhalative Verabreichung des zweiten Wirkstoffgemisches in einer therapeutisch wirksamen Dosierung über einen Zeitraum von jeweils ca. 30 Minuten.

Diese Ausführungsform hat den Vorteil, dass die pharmazeutische Zusammensetzung in einem zeitlich bestimmten Behandlungsplan verabreicht wird, der sich als besonders wirkungsvoll bei der Behandlung von Atemwegserkrankungen herausgestellt hat.

Die für die erfindungsgemäße pharmazeutische Zusammensetzung beschriebenen Merkmale, Eigenschaften und Vorteile gelten für die erfindungsgemäße Verwendung und für das erfindungsgemäße Kit entsprechend.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nun anhand von Ausführungsbeispielen näher beschrieben, aus denen sich weitere Merkmale, Eigenschaften und Vorteile ergeben. Die Ausführungsbeispiele sind rein illustrativ und schränken die Reichweite der Erfindung nicht ein.

Es wird Bezug genommen auf die beigefügte Figur, in der Folgendes dargestellt ist.

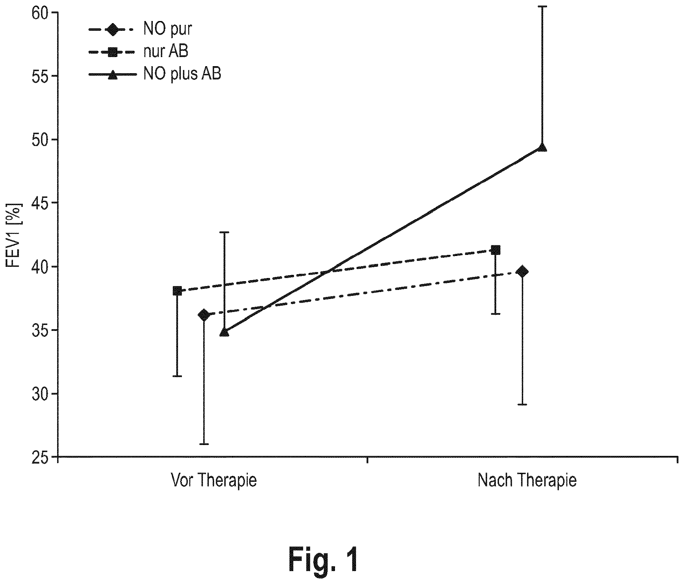

Fig. 1 demonstriert anhand des FEV₁ Wertes die Verbesserung der Lungenfunktion bei der Anwendung der erfindungsgemäßen Zusammensetzung im Vergleich zu einer isolierten Antibiotika-Therapie und zu einer NO Monotherapie.

### Ausführungsbeispiele

### 1. Vorarbeiten: Isolierte NO-Therapie

In einer ersten klinischen Studie wurden acht erwachsene CF-Patienten mit bereits eingeschränkter Lungenfunktion, mit einem FEV₁ Wert von < 80% und > 30% (im Durchschnitt FEV₁ < 50%), mit gasförmigem NO behandelt. Die Patienten erhielten gasförmiges NO in einer Konzentration von 160 ppm dreimal am Tag innerhalb von zwei Behandlungs-Perioden von jeweils 5 Tagen über zwei aufeinanderfolgende Wochen. Jede einzelne Behandlung dauerte 30 min, wobei zwischen den Behandlungen eine Pause von 3 Stunden eingehalten wurde. Der detaillierte Studienverlauf ist in der Veröffentlichung Deppisch, C., Herrmann, G., Graepler-Mainka, U. et al. Infection (2016) 44: 513 beschrieben.

Dabei zeigte sich, dass die Inhalation von gasförmigem Stickstoff von den Patienten gut toleriert wurde. Ferner zeigte sich, dass eine absolute signifikante Lungenfunktionszunahme von im Mittel FEV₁ + 8,7% (17,2% relativ) zum Ausgangswert erreicht werden konnte. Ferner wurde ein antibakterieller als auch ein antifugaler Effekt nachgewiesen. Eine Zusammenfassung der Ergebnisse ist in der nachstehenden Tabelle 1 dargestellt.

**Tab. 1: Sekundäre Parameter vor und nach der Behandlung mit gasförmigem NO (n=8).**

| | FVC [%] | | FEV₁ [%] | | MEF ₂₅₋₇₅ [%] | |
|---|---|---|---|---|---|---|
| | vor | nach | vor | nach | vor | nach |
| MW | 63,3 | 75,6 | 49,9 | 58,7 | 28,6 | 36,7 |
| SD [±] | 13,0 | 14,6 | 11,2 | 14,3 | 15,6 | 18,6 |
| p-Wert | | 0,012 | | 0,012 | | 0,036 |

Innerhalb dieser Studie zeigte sich ferner (nicht veröffentlicht), dass in Patienten mit einem eingeschränkten Lungenvolumen (FEV₁ < 50%) aufgrund des protrahierenden destruktiven Umbaus der Lunge nicht die positiven Ergebnisse erzielt werden wie in Patienten mit einem FEV₁ > 50%. Die Erfinder nahmen daher an, dass die nicht mehr belüfteten Areale der fortgeschrittenen CF-Lungenerkrankung durch das NO-Gas nicht mehr erreicht werden und somit das Gas eine nicht mehr ausreichend suffiziente antimikrobielle und damit (indirekte) antiinflammatorische Wirkung liefert.

### 2. NO-Therapie bei CF-Patienten mit einem FEV₁ < 50%

Mit den gewonnenen Erkenntnissen aus den Vorarbeiten wurde im nächsten Schritt untersucht, welchen Einfluss eine isolierte NO Therapie auf CF-Patienten mit einem geringeren FEV₁ Wert hat.

Sieben erwachsene Patienten, mit einem FEV₁ Mittelwert von 36 ± 11%, wurden analog zu der unter Punkt 1 gezeigten Therapie in Einzelversuchen behandelt. Dazu wurde ihnen 160 ppm NO dreimal für jeweils 30 min täglich für 10 bis 15 Tage (MW 12,7 ± 1,9 Tage) verabreicht. Das Alter der Patienten lag im Durchschnitt bei 28,9 ± 6,9 Jahren. Die Lungenfunktion betrug initial FEV₁ 36,2 ± 10,6% und nach der Therapie 39,6 ± 10,9%, Δ 3,4 ± 2,8%, p = 0,009. Die Patienten erhielten nebenher weiterhin ihre übliche Routinetherapie (Sekretolyse, inhalative Antibiotika wie Tobramycin, Aztreonam oder Colistin im Rahmen einer Dauertherapie etc.).

Vier Patienten wiesen eine chronische *Pseudomonas* Infektion auf. Dagegen wiesen drei Patienten eine pathogene Besiedelung mit *Staphylokokken* und zwei Patienten eine solche mit *Stenotrophomonas.* Zusätzlich wurden bei fünf Patienten das Bakterium *Candida* und bei vier Patienten *Aspergillus* nachgewiesen. Ein Patient wies zusätzlich eine M. *abscessus*-Infektion auf.

Eine Zusammenfassung der Ergebnisse ist in Tabelle 2 dargestellt.

Innerhalb der Studie zeigte sich nur noch eine signifikante FEV₁ Zunahme von 3,4% absolut und 10% relativ zum Ausgangswert. Diese Ergebnisse bestätigten die zuvor angestellten Hypothesen, dass eine isolierte NO-Therapie nicht genügend wirkungsvoll bei Patienten mit einer stark eingeschränkten Lungenfunktion ist.

### 3. Klinische Studie mit der erfindungsgemäßen pharmazeutischen Zusammensetzung

In einer weiteren klinischen Studie wurde im Rahmen einer akuten antibakteriellen Interventionstherapie ein Patient mit einem FEV₁ von < 40% erstmals mit der erfindungsgemäßen pharmazeutischen Zusammensetzung über 14 Tage behandelt. Dabei wurde dem Patienten zum einen innerhalb einer klassischen intravenösen antibiotischen Therapie Meropenem und Tobramycin verabreicht. Ferner wurde der Patient mit NO behandelt. Der Patient erhielt außerdem weiterhin seine übliche Routinetherapie (Sekretolyse, inhalative Antibiotika wie Tobramycin, Aztreonam oder Colistin im Rahmen einer Dauertherapie etc.), sodass der Patient zum einen invasiv als auch inhalativ mit Antibiotika behandelt wurde. Diese antibiotische Behandlung entspricht der Verabreichung des ersten Wirkstoffgemisches der erfindungsgemäßen pharmazeutischen Zusammenfassung, welches zumindest drei Antibiotika enthält.

Während dieser Einzelstudie wurde eine überraschend deutliche FEV₁ Zunahme gemessen, die weit über das bis dato bei dem Patienten gemessene Niveau hinausging (FEV₁ von 38% auf 54%, Höchstwert der letzten zwei Jahr war maximal 45%).

Aufgrund dieses synergistischen Effektes wurden in einem nächsten Schritt weitere Einzelversuche durchgeführt, um die beobachteten Effekte zu reproduzieren.

Die Folgestudie wurde bei zehn CF-Patienten mit einem FEV₁ Mittelwert von 35,0% durchgeführt. Dazu wurden sie in Einzelversuchen behandelt. Das Alter der Patienten lag im Durchschnitt bei 19,4 ± 10,9 Jahre. Die Lungenfunktion betrug initial FEV₁ 35,0 ± 7,5% und nach der Therapie 49,4 ± 13,5%, Δ 14,5 ± 7,9%, p = 0,0001. Hierbei wurden ihnen 160 ppm NO dreimal für jeweils 30 min täglich für 8 bis 18 Tage (MW 11,3 ± 4 Tage) verabreicht. Auch diese Patienten wurden nebenher weiterhin im Rahmen ihrer üblichen Routinetherapie behandelt.

Drei Patienten zeigten eine chronische Infektion mit *Pseudomonas,* drei Patienten zeigten eine Infektion mit *Staphylokokken* und fünf Patienten eine Infektion mit *Stenotrophomo*nas. Zusätzlich wurden bei fünf Patienten *Candida* Bakterien nachgewiesen. Ein Patient war zusätzlich mit *MRSA* infiziert.

Neun von zehn Patienten wurden mit 100 mg/kg KG Meropenem (3 ED) intravenös und alle Patienten mit 8 mg/ kg KG Tobramycin (1 ED) intravenös therapiert. Ein Patient wurde mit intravenösem Ceftazidim 200 mg/kg KG (3 ED) und intravenösem Tobramycin behandelt. Drei von zehn Patienten wurden zusätzlich zu Meropenem und Tobramycin auch Ceftazidim verabreicht. Einem Patienten wurde zusätzlich zu Meropenem und Tobramycin auch 400 mg/kg KG Fosfomycin intravenös verabreicht.

Innerhalb dieser Studie zeigte sich eine hochsignifikante absolute Verbesserung der Lungenfunktion um überraschende 14,5%, die sich als eine relative Verbesserung von 41% berechnen lässt.

Eine Zusammenfassung der Ergebnisse ist in Tabelle 2 dargestellt.

**Tab. 2: Sekundäre Parameter der unterschiedlichen Therapieformen (isolierte antibiotische Kombinationstherapie (Punkt 4), isolierte inhalative NO-Therapie (Punkt 2), erfindungsgemäße Therapie (Punkt 3)) vor und nach der Behandlung.**

| | | FVC [%] | | Diff. [%] | | FEV₁ [%] | | Diff. [%] | | MEF₂₅₋₇₅ [%] | | Diff. [%] | |
|---|---|---|---|---|---|---|---|---|---|---|---|---|---|
| | | vor | nach | abs. | rel. | vor | nach | abs. | rel. | vor | nach | abs. | rel. |
| Therapie gemäß Punkt 4 | MW | 56,6 | 60,5 | 3,8 | 7,0 | 37,8 | 41,5 | 3,7 | 10,6 | 13,3 | 15,6 | 2,3 | 22,0 |
| n=8 | SD [±] | 7 | 5 | 5 | 10 | 6 | 5 | 2 | 6 | 7 | 8 | 3 | 24 |
| p-Wert | | | 0,04 | | | | 0,0001 | | | | 0,03 | | |
| Therapie gemäß Punkt 2 | MW | 44,3 | 47,0 | 2,7 | 7,9 | 36,2 | 39,6 | 3,4 | 9,9 | 22,6 | 30,3 | 7,7 | 37,2 |
| n=7 | SD [±] | 10 | 6 | 5 | 11 | 11 | 11 | 3 | 8 | 17 | 21 | 7 | 26 |
| p-Wert | | | 0,11 | | | | 0,009 | | | | 0,02 | | |
| Erfindungsgemäße Therapie gemäß Punkt 3 | MW | 57,3 | 71,9 | 14,6 | 28,5 | 35,0 | 49,4 | 14,5 | 41,2 | 14,3 | 18,1 | 3,8 | 34,7 |
| | SD [±] | 12 | 11 | 8 | 19 | 8 | 14 | 8 | 17 | 10 | 9 | 3 | 34 |
| | | | 0,0002 | | | | 0,0001 | | | | 0,002 | | |

### 4. Kontrolle durch invasive antibiotische Therapie

Um die gewonnenen Ergebnisse der erfindungsgemäßen Therapie nicht nur mit einer isolierten NO-Therapie vergleichen zu können, sondern auch mit einer isolierten antibiotischen Therapie, wurden zusätzlich die Ergebnisse aus zuvor durchgeführten invasiven antibiotischen Therapien betrachtet. Hierzu wurden bei acht der zehn Patienten, welche erfindungsgemäß therapiert wurden (unter Punkt 3), retroperspektiv durchgeführte intravenös antibiotische Therapien analysiert.

Die ausgewählten Therapien erfolgten über 14 Tage. Das Alter der Patienten lag im Durchschnitt bei 22,5 ± 8,4 Jahren. Die Lungenfunktion betrug initial FEV₁ 37,8 ± 6,2% und nach der Therapie 41,5 ± 5,1%, Δ 3,7 ± 1,9%, p = 0,0005. Die Patienten wurden auch nebenher im Rahmen ihrer üblichen Routinetherapie behandelt.

Fünf Patienten zeigten eine chronische Infektion mit *Pseudomonas,* vier Patienten zeigten eine Infektion mit *Staphylokokken* und vier Patienten eine Infektion mit *Stenotrophomo*nas. Zusätzlich wurden bei fünf Patienten *Candida* und bei einem Patienten *Aspergillus* Bakterien nachgewiesen.

Allen acht Patienten wurde 100 mg/kg KG Meropenem (3 ED) intravenös und 8 mg/kg KG Tobramycin (1 ED) intravenös verabreicht. Zwei Patienten bekamen zusätzlich intravenös Ceftazidim 200 mg/ kg KG (3 ED).

Innerhalb dieser Studie zeigte sich im Vergleich zu einer isolierten NO-Therapie eine signifikante FEV₁ Zunahme zum Ausgangswert von im Mittel FEV₁ 3,7% absolut. Dies entspricht einer relativen Zunahme von 11,0%.

Vergleiche der FEV₁-Werte der isolierten antibiotischen Kombinationstherapie (Punkt 4), der isolierte inhalative NO-Therapie (Punkt 2) und der erfindungsgemäße Therapie (Punkt 3) sind aus dem Graphen in Figur 1 zu entnehmen. Dort sind die FEV₁-Werte der einzelnen Therapien vor und nach der Behandlung gegeneinander aufgetragen.

### 5. Fazit

Die Erfinder konnten eindrucksvoll anhand von klinischen Studien demonstrieren, dass durch die Kombination von einem Wirkstoffgemisch aus zumindest drei Antibiotika und einem Wirkstoffgemisch aus gasförmigen NO aufgrund einer synergistischen Wirkung effektiv Atemwegserkrankungen behandelt werden können.

Sowohl die isolierte invasiv antibiotische- als auch die isolierte inhalative NO-Therapie ist bei mittelschwer bis schwer betroffenen CF-Patienten hingegen nur kurzfristig in der Lage die Lungenfunktion zu verbessern. Die Lebensqualität und ggf. die Lebenserwartung der Patienten wird dadurch nicht zufriedenstellend verbessert. Dabei besteht kein statistischer Unterschied (p = 0.41) zwischen den beiden isolierten Therapien. Die isolierte NO Therapie ist der bis dato routinemäßig durchgeführten invasiven antibiotischen Therapie in der Wirkung weder unter- noch überlegen.

Die Erfinder konnten zeigen, dass die Kombination dieser beiden Therapien nicht nur einen additiven sondern einen synergistischen Effekt zeigt. Dieser zeigt sich in einem hochsignifikanten Unterschied in der Lungenfunktion (am Beispiel des FEV₁ Wertes) zwischen der erfindungsgemäßen Therapie und den jeweils isolierten Therapien. Von dieser synergistischen Wirkung war bis dato nicht auszugehen.

Ferner konnten die Erfinder zeigen, dass die erfindungsgemäße Therapie auch langfristig einen positiven Effekt zeigt. Mit dieser neuen erfindungsgemäßen Therapie ist es außerdem gelungen, eine bis dato nicht mehr zu erwartende Lungenfunktionsverbesserung zu erzielen.

Die erfindungsgemäße Therapie lässt sich auf weitere Atemwegserkrankungen wie die COPD übertragen.

## Patentansprüche

1. Pharmazeutische Zusammensetzung zur Anwendung bei der Behandlung einer Atemwegserkrankung, mit mindestens zwei gegebenenfalls räumlich getrennten Wirkstoffgemischen, wobei das erste Wirkstoffgemisch zumindest drei Antibiotika aufweist und das zweite Wirkstoffgemisch zumindest gasförmiges NO aufweist.

2. Pharmazeutische Zusammensetzung nach Anspruch 1, **dadurch gekennzeichnet, dass** die Antibiotika des ersten Wirkstoffgemisches ausgewählt sind aus der Gruppe bestehend aus: Cephalosporine, Aminoglycoside, Makrolide, Polymyxine, Peneme, Carbapenem, Penicilline, Epoxyd-Antibiotika, einschließlich Chinolone.

3. Pharmazeutische Zusammensetzung nach Anspruch 1 oder 2, **dadurch gekennzeichnet, dass** zumindest ein Antibiotikum des ersten Wirkstoffgemisches ein Aminoglycosid ist.

4. Pharmazeutische Zusammensetzung nach Anspruch 3, **dadurch gekennzeichnet, dass** das Aminoglycosid Tobramycin ist.

5. Pharmazeutische Zusammensetzung nach einem der vorherigen Ansprüche, **dadurch gekennzeichnet, dass** das erste Wirkstoffgemisch für eine Applikation im Wege einer intravenösen und/oder inhalativen und/oder oralen Verabreichung ausgestaltet ist.

6. Pharmazeutische Zusammensetzung nach einem der vorherigen Ansprüche, **dadurch gekennzeichnet, dass** das zweite Wirkstoffgemisch für eine Applikation im Wege einer Inhalation ausgestaltet ist.

7. Pharmazeutische Zusammensetzung nach einem der vorherigen Ansprüche, **dadurch gekennzeichnet, dass** das gasförmige NO des zweiten Wirkstoffgemisches inhalativ in einer Konzentration von ca. 120 bis ca. 180 ppm, vorzugsweise von ca. 140 bis ca. 170 ppm, insbesondere ca. 160 ppm verabreicht wird.

8. Pharmazeutische Zusammensetzung nach einem der vorherigen Ansprüche, **dadurch gekennzeichnet, dass** die Atemwegserkrankung ausgewählt ist aus der Gruppe bestehend aus: Cystischer Fibrose (CF), chronisch obstruktiver Bronchitis (COPD), non-CF Bronchiektasien, Ventilator-assoziierte Pneumonie (VAP), Bronchiolitis, Pharyngitis, Laryngitis, Tracheitis, Pneumonie, Sinusitis, Asthma, und primäre ciliäre Dyskinesie (PCD).

9. Pharmazeutische Zusammensetzung nach einem der vorherigen Ansprüche, **dadurch gekennzeichnet, dass** die Atemwegserkrankung eine bakterielle, eine mykobakterielle oder fungizide Atemwegserkrankung ist.

10. Pharmazeutische Zusammensetzung nach Anspruch 9, **dadurch gekennzeichnet, dass** die bakterielle Atemwegserkrankung Bakterien involviert, die ausgewählt sind aus der Gruppe bestehend aus: *Pseudomonas aeruginosa*, multiresistenter *Staphylococcus aureus, Staphylococcus aureus, Haemophilus influenzae* (HI) b, nicht-typisierbarer HI (NTHI), *Moraxella catarrhalis*, *Streptococcus pneumonia, Burkholderia cepacia, Acinetobacter baumanii,* strikt-anaerobe Bakterien, einschließlich *Prevotella spp.*

11. Pharmazeutische Zusammensetzung nach einem der vorherigen Ansprüche, **dadurch gekennzeichnet, dass** die Behandlung einer Atemwegserkrankung an Patienten mit eingeschränkter Lungenfunktion mit einem FEV₁ Wert < 80%, vorzugsweise < 50%, insbesondere < 40% erfolgt.

12. Pharmazeutische Zusammensetzung nach einem der vorherigen Ansprüche, **dadurch gekennzeichnet, dass** die pharmazeutische Zusammensetzung nach einem Behandlungsplan verabreicht wird, der einen oder mehrere Zyklen umfasst, wobei jeder Zyklus aus den folgenden Schritten besteht:
(a) mindestens einmal tägliche Verabreichung des ersten Wirkstoffgemisches in einer therapeutisch wirksamen Dosierung, wobei die Verabreichung inhalativ, oral und/oder invasiv durchgeführt wird, und
(b) zwei- bis dreimal tägliche inhalative Verabreichung des zweiten Wirkstoffgemisches in einer therapeutisch wirksamen Dosierung über einen Zeitraum von jeweils ca. 30 Minuten.

13. Verwendung einer pharmazeutischen Zusammensetzung zur Behandlung einer Atemwegserkrankung, mit mindestens zwei gegebenenfalls räumlich getrennten Wirkstoffgemischen, wobei das erste Wirkstoffgemisch zumindest drei Antibiotika aufweist und das zweite Wirkstoffgemisch zumindest gasförmiges NO aufweist.

14. Kit zur Behandlung einer Atemwegserkrankung, aufweisend:
- ein erstes Wirkstoffgemisch von zumindest drei Antibiotika, und
- ein zweites Wirkstoffgemisch von zumindest gasförmigem NO.
